# EUROPEAN PATENT APPLICATION

(11) **EP 1 905 770 A1**
(43) Date of publication of application: **02.04.2008**
(21) Application number: 07113477.9
(22) Date of filing: 31.07.2007
(51) Int. Cl.: C07D 405/14

(54) **A process for the preparation of phenyltetrazole compounds**

(30) Priority: 27.09.2006 IT MI20061848
(71) Applicant: Dipharma Francis S.r.l., 20021 Baranzate (MI) (IT)
(72) Inventor: Razzetti, Gabriele, 20099, SESTO S. GIOVANNI (IT); Colombo, Lino, 27100, PAVIA (IT); Rota, Paola, 20078, SAN COLOMBANO AL LAMBRO (IT); Allegrini, Pietro, 20097, SAN DONATO MILANESE (IT)
(74) Representative: Minoja, Fabrizio

(57) **Abstract**

A process for the preparation of olmesartan medoxomil, and derivatives thereof, by use of novel phenyltetrazole intermediates.

## Description

### FIELD OF THE INVENTION

The present invention relates to a novel process for the preparation of olmesartan medoxomil, derivatives thereof and novel phenyltetrazole compounds useful as intermediates in the preparation thereof.

### TECHNOLOGICAL BACKGROUND

Olmesartan medoxomil, namely (5-methyl-2-oxo-1,3-dioxolen-4-yl)methyl-4-(1-hydroxy-1-methylethyl)-2-propyl-1-[4-[2-(tetrazol-5-yl)phenyl]-phenyl}methylimidazole-5-carboxylate, of formula (**A**) 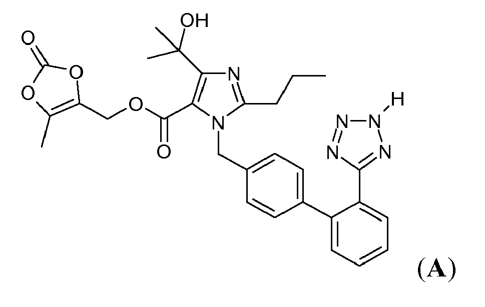 is known from EP 0503785. Olmesartan medoxomil is a "prodrug" of olmesartan, a medicament active as an angiotensin II inhibitor, useful in the treatment of hypertension, anxiety, glaucoma and cardiac attacks. In view of its therapeutical importance, there is the need for alternative synthetic methods providing highly pure and bioavailable olmesartan with reduced production costs.

### SUMMARY OF THE INVENTION

A particularly efficient alternative process for the preparation of olmesartan medoxomil has now been found, which comprises the reaction of a compound of formula (**III**) with a compound of formula (**II**) or (**IV**), as herein defined, and the removal of the tetrazole nitrogen-protecting group from the resulting intermediate. The process of the invention surprisingly allows the selective removal of the tetrazole nitrogen-protecting group without inducing the simultaneous hydrolysis of the medoxomil group, which is unexpectedly unaffected during the coupling reaction according to alternative a) of the process herein described.

### DETAILED DISCLOSURE OF THE INVENTION

An object of the invention is a process for the preparation of a compound of formula (**I**) 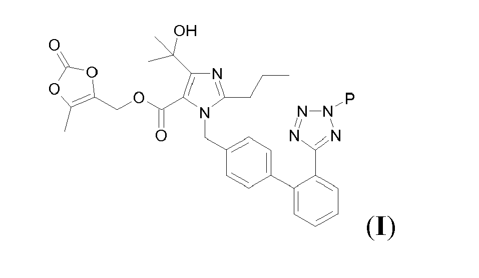 wherein P is a hydrogen atom or a 1-methyl-1-phenylethyl group, comprising:
a) the reaction of a compound of formula (**II**), or a salt thereof, 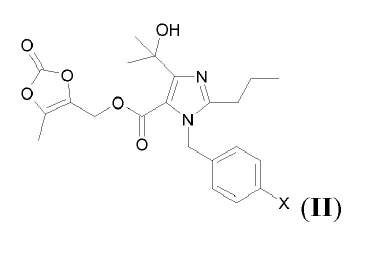 wherein X is a leaving group, with a synthon of formula (**III**), or a salt thereof 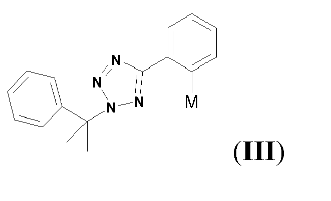 wherein
   M is a -B(OR₁OR₂) group wherein each of R₁ and R₂ is, independently, hydrogen, C₁-C₈ alkyl, aryl, aryl-C₁-C₈ alkyl or R₁ and R₂, taken together, form a -(CH₂)ₘ-V-(CH₂)ₙ group, wherein m and n, which can be the same or different, are 0 or 1, and V is NR₃ or C(R₃)₂ wherein R₃ is hydrogen, C₁-C₈ alkyl, aryl or aryl-C₁-C₈ alkyl; or M is a lithium or copper atom or a halogenated metal;
   in the presence of a catalyst, an organic ligand and a basic agent; or
b) the reaction of a synthon of formula (**III**), or a salt thereof, as defined above, with a compound of formula (**IV**), or a salt thereof, 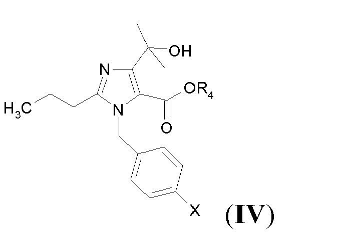 wherein X is a leaving group, and R₄ is C₁-C₈ alkyl, aryl or aryl-C₁-C₈ alkyl;
   in the presence of a catalyst, an organic ligand and a basic agent;
   to obtain a compound of formula (**V**) 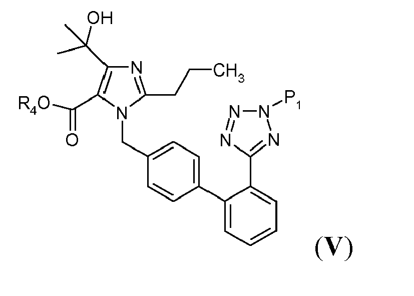 wherein P₁ is a 1-methyl-1-phenylethyl group and R₄ is as defined above;
b') the subsequent hydrolysis of the heterocycle ester group in a compound of formula (**V**), as defined above,
   to obtain a compound of formula (**VI**), or a salt thereof, 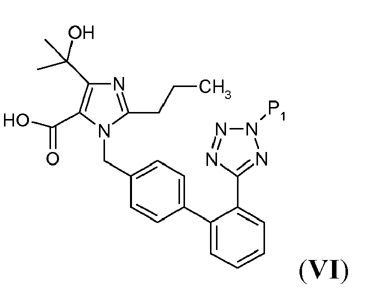 wherein P₁ is as defined above;
b") the subsequent reaction of a compound of formula (**VI**), as defined above, or a salt thereof, with a compound of formula (**VII**) 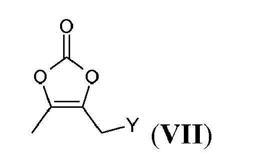 wherein Y is halogen; and, if desired, the conversion of a compound of formula (**I**) to another compound of formula (**I**).

A salt of a compound of formula (**II**), (**III**), (**IV**) or (**VI**) is for example a pharmaceutically acceptable salt, typically the sodium, potassium, magnesium or calcium salt, or a salt with a hydrohalo acid, such as hydrochloric or hydrobromic acid, in particular sodium or potassium.

A leaving group X is typically a halogen atom, such as chlorine, bromine or iodine, in particular bromine; or a hydroxy group activated by esterification, for example with an alkanesulfonate group, typically methanesulfonyloxy, toluenesulfonyloxy, fluorosulfonyloxy, trifluoromethanesulfonyloxy or nonafluorobutanesulfonyloxy. The leaving group X is preferably bromine.

M as a halogenated metal is e.g. a zinc, magnesium, nickel, copper or boron halide; preferably -ZnCl, -MgCl, -NiCl, -CuCl, -BCl₂, - ZnBr, -MgBr, -CuBr, and -BBr₂; more preferably ZnCl.

R₁, R₂, R₃ and/or R₄ as a C₁-C₈ alkyl group or residue, which can be straight or branched, are preferably C₁-C₄ alkyl; in particular methyl, ethyl, propyl, isopropyl, butyl, sec-butyl or tert-butyl.

R₁, R₂, R₃ and/or R₄ as an aryl group are for example phenyl or naphthyl, preferably phenyl.

R₁, R₂, R₃ and/or R₄ as an aryl-C₁-C₈ alkyl group are preferably a benzyl or phenylethyl group.

M is preferably a -B(OR₁OR₂) group wherein each of R₁ and R₂ is, independently, hydrogen or C₁-C₄ alkyl, in particular hydrogen.

A catalyst is typically a Pd, Pt or Ni salt, such as a chloride, bromide, iodide, acetate, acetylacetonate, carbonate, hydroxide; preferably is a palladium salt, more preferably a palladium (**II**) salt, in particular palladium (**II**) acetate.

An organic ligand is typically a phosphine, such as tricyclohexylphosphine, triphenylphosphine, tris-(3-hydroxypropyl)-phosphine, tributylphosphine, dppb (1,4-bis(diphenylphosphino)-butane), or dppf (diphenylphosphineferrocene), preferably triphenylphosphine.

A basic agent can be an organic base, such as a straight or branched tertiary amine, typically triethylamine; or an inorganic base, such as potassium, sodium or cesium carbonate, sodium or potassium acetate, sodium or potassium hydroxide, sodium or potassium phosphate, and sodium or potassium hydrogen phosphate; preferably potassium carbonate.

Y is for example chlorine, bromine or iodine, in particular chlorine.

The molar ratio of basic agent to compound of formula (**II**) or (**IV**), or a salt thereof, approximately ranges from 1:1 to 4:1, preferably approximately from 1:1 to 2:1.

The reaction between a compound of formula (**III**), or a salt thereof, and a compound of formula (**II**), or a salt thereof, or of formula (**IV**), or a salt thereof, can be carried out in the presence of an organic solvent, or in a mixture of two or three organic solvents; or in a mixture of one, two or three of them with water. An organic solvent is typically an aromatic hydrocarbon, e.g. toluene, xylene; or an ether, e.g. tetrahydrofuran, methyl-tetrahydrofuran, dioxane; or an ester, e.g. ethyl acetate or butyl acetate; or a dipolar aprotic solvent, e.g. dimethylformamide, dimethylacetamide, dimethylsulfoxide or N-methylpyrrolidone; or an alkanol, e.g. a C₁-C₄ alkanol, preferably methanol, ethanol or isopropanol.

The reaction is preferably carried out in a tetrahydrofuran-water mixture.

The reaction can be carried out at a temperature approximately ranging from 0°C to the reflux temperature of the reaction mixture, preferably approximately from 30°C to the reflux temperature, more preferably from 50 to 80°C.

According to a particularly preferred aspect, the reaction can be carried out between a compound of formula (**II**) or (**IV**), wherein X is bromine, and a synthon of formula (**III**), wherein M is a group-B(OR₁OR₂) wherein each of R₁ and R₂ is hydrogen; in the presence of palladium (II) acetate, triphenylphosphine, potassium carbonate, in a tetrahydrofuran-water mixture.

The hydrolysis of the ester group in a compound of formula (**V**) to obtain an acid of formula (**VI**), as well as the introduction of the medoxomil group by reaction of a compound of formula (**VI**) with a compound of (**VII**), can be carried out according to known methods. A compound of formula (**I**) can be converted to another compound of formula (**I**) according to known methods. For example, the removal of the protecting group in a compound of formula (**I**), wherein P is a 1-methyl-1-phenylethyl group, to obtain a corresponding compound of formula (**I**) wherein P is hydrogen (olmesartan medoxomil), can be carried out according to EP 1555260. Following this procedure, for example operating in an approximately 37% hydrochloric acid aqueous solution, the cumyl group is selectively removed, while the medoxomil group is surprisingly and unexpectedly unaffected during the coupling reaction of the alternative a) of the process.

Synthons of formula (**III**) are known or can be obtained according to known methods, for example as disclosed in WO 2005/014560.

The compounds of formula (**I**) wherein P is a 1-methyl-1-phenylethyl group, of formulae (**II**), (**V**) and (**VI**), as herein defined, or a salt thereof, are novel and are a further object of the invention.

Preferred compounds are the compounds of formula (**II**) and (**IV**), wherein X is halogen, in particular bromine. These compounds can be obtained according to known methods, for example a compound of formula (**II**) or of formula (**IV**) in which X is a leaving group, can be obtained by reaction between a compound of formula (**VIII**) or (**IX**), respectively, 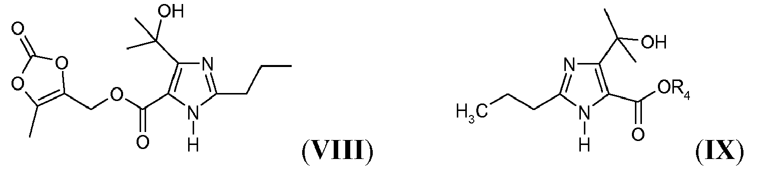 with a compound of formula (**X**) 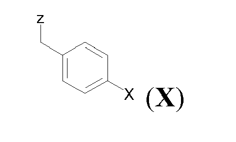 wherein each of X and Z, which can be the same or different, is a leaving group as defined above, in the presence of a basic agent.

A basic agent can be an organic or inorganic base, as exemplified above, preferably potassium carbonate.

The reaction can be carried out in the presence of an organic solvent, typically an aromatic hydrocarbon, e.g. toluene, xylene; or an ether, e.g. tetrahydrofuran, dioxane; or an ester, e.g. ethyl acetate or butyl acetate; or a chlorinated solvent, e.g. dichloromethane, or an alkanol, such as methanol, ethanol or isopropanol; or a dipolar aprotic solvent, such as dimethylformamide, dimethylacetamide, N-methyl pyrrolidone, dimethylsulfoxide, preferably dimethylacetamide.

The reaction can be carried out at a temperature approximately ranging from 0°C to the reflux temperature of the reaction mixture, preferably from 20 to 30°C.

Alternatively, a compound of formula (**II**) can be prepared starting from a compound of formula (**IV**), or a salt thereof, by a process comprising the hydrolysis of the ester in said compound to obtain a compound of formula (**XI**), or a salt thereof, 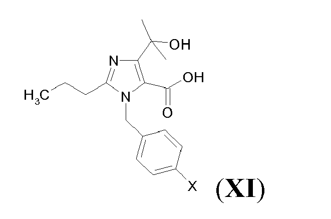 wherein X is defined as above, and the subsequent reaction with a compound of formula (**VII**), as defined above, according to the methods reported above.

The compounds of formula (**IV**) and formula (**XI**), as herein defined, can be represented as a compound of formula (**XII**) 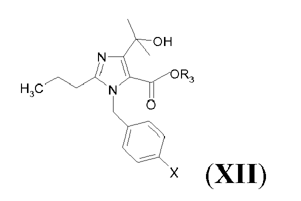 wherein R₃ and X are as defined above. The compounds of formula (**XII**), and the salts thereof, are novel compounds and are a further object of the invention. A salt of a compound of formula (**XII**) is for example a pharmaceutically acceptable salt, as defined above.

The compounds of formulae (**VII**), (**VIII**), (**IX**) and (**X**) are either known, or can be obtained with known methods or are commercially available.

A compound of formula (**I**) thus obtained, in particular wherein P is a hydrogen atom, has purity equal to or higher than 99.5%, typically higher than 99.9%; any impurities detectable according to conventional analytic techniques are anyway equal to or lower than 0.1%. Said compound usually has a particulate having a D[4,3] mean diameter approximately ranging from 40 to 250 µm, typically from 50 to 150 µm. If desired, the mean diameter can be reduced according to known methods, typically by fine grinding, thereby obtaining a product with a mean diameter lower than 40 µm, preferably ranging from 1 to 20 µm.

The following examples illustrate the invention.

### EXAMPLE 1: 3-(4-Bromo-benzyl)-5-(1-hydroxy-1-methyl-ethyl)-2-propyl-3H-imidazol-4-carboxylic acid ethyl ester (IV; X=Br)

NaH (228 mg, 9.53 mmol), previously placed under N₂ atmosphere and washed with pentane to remove paraffin, is reacted at 0°C with a solution of a compound of formula (**IX**), wherein R₄ is ethyl (1.76 g, 7.33 mmol), dissolved in DMF (5 mL). 10 minutes after the addition, a solution of a compound of formula (X), wherein X=Z=Br (2.02 g, 8.06 mmol) in DMF (10 mL) is added thereto. The mixture is left under stirring for 1 hour, then diluted with ethyl acetate and water. The organic phase is separated, dried and evaporated to a residue. The crude is subjected to purification by flash chromatography with a 50 mm diameter column, eluting with a hexane/ethyl acetate 1:1 mixture. 2.15 g of the title compound are obtained; yield: 45%.

¹H-NMR (CDCl₃) 0.96 (3H, t, J=7.4 Hz) 1.18 (3H, t, J=7.1 Hz) 1.65 (6H, s) 1.70 (2H, sx, J=7.5 Hz) 2.62 (2H, t, J=7.7 Hz) 4.23 (2H, q, J=7.1 Hz) 5.41 (2H, s) 5.76 (1H, s) 6.81 (2H, d, J=8.3 Hz) 7.46 (2H, d, J=8.3 Hz).

### EXAMPLE 2: 3-(4-Bromo-benzyl)-5-(1-hydroxy-1-methyl-ethyl)-2-propyl-3H-imidazol-4-carboxylic acid ethyl ester (IV; X=Br)

A mixture of a compound of formula (**IX**), in which R₄ is ethyl (100 g, 0.417 mol), a compound of formula (**X**), in which X=Z=Br (107.5 g, 1.03 mol) and K₂CO₃ (71 g, 0.516 mol) in DMA (400 mL) is reacted at room temperature, under stirring, for 18 hours. The reaction mixture is then diluted with water and the precipitated solid is filtered and dried in a static dryer under vacuum.

152 g of the title compound are obtained; yield: 89%.

### EXAMPLE 3: 5-(1-Hydroxy-1-methyl-ethyl)-3-{2-[2-(1-methyl-1-phenylethyl)-2H-tetrazol-5-il]-biphenyl-4-yl-methyl}-2-propyl-3H-imidazol-4-carboxylic acid ethyl ester (V)

2-(2-Cumyltetrazolyl)phenylboronic acid (1.13 g, 3.66 mmol) is suspended in toluene under N₂ atmosphere; water is added (60 µL) and the mixture is left under stirring at room temperature for 30 min, after which it is added with more water (60 µL), then with K₂CO₃ (776 mg, 5.612 mmol) and the compound of formula **(IV)** obtained in example 1 (1.0 g, 2.44 mmol). The mixture is left under stirring at room temperature for 30 min, then added with the catalyst solution previously prepared according the following procedure.

Catalyst preparation: P(Ph)₃ (409 mg, 1.562 mmol) is dissolved in THF under inert N₂ atmosphere, then added with Pd(OAc)₂ (48.85 mg, 0.219 mmol) and the resulting solution is adjusted to 60°C, left at this temperature for 30 min, then cooled to room temperature. The reaction mixture is kept at room temperature for about 5 hours, when the complete disappearance of the starting compound of formula (**IV**) is observed. After that, the solvent is evaporated off under reduced pressure, the residue is taken up with H₂O and extracted with ethyl acetate. The organic phase is dried over Na₂SO₄, filtered and the solvent is evaporated off under reduced pressure. The reaction crude is subjected to purification by flash chromatography on a 50 mm diameter column eluting with a hexane/ethyl acetate 6:4 mixture and subsequently hexane/ethyl acetate 1:1 mixture. 1.30 g of crude are obtained which are washed with 1 M NaOH. The reaction product is extracted with Et₂O, the organic phase is dried with Na₂SO₄, and the solvent is evaporated off under reduced pressure.

954 mg of the title compound are obtained; yield: 66%.

¹H-NMR (CDCl₃) 0.96 (3H, t, J=7.4 Hz) 1.18 (3H, t, J=7.1 Hz) 1.66 (6H, s) 1.72 (2H, sx, J=7.5 Hz) 2.03 (6H, s) 2.64 (2H, br) 4.23 (2H, q, J=7.1 Hz) 5.45 (2H, s) 5.76 (1 H, s) 6.81 (2H, d, J=8.3 Hz) 7.46 (2H, d, J=8.3 Hz) 7.87-6.83 (13H, m).

### EXAMPLE 4: 5-(1-Hydroxy-1-methyl-ethyl)-3-{2-[2-(1-methyl-1-phenylethyl)-2H-tetrazol-5-yl]-biphenyl-4-yl-methyl}-2-propyl-3H-imidazol-4-carboxylic acid ethyl ester (V)

The product of formula (**IV**) of Example 1 (30 g, 73.5 mmoles), 2-(2-cumyltetrazolyl)-phenylboronic acid (26 g, 84.5 mmoles), K₂CO₃ (20.32 g, 147 mmoles), THF (120 mL) and H₂O (20 mL) are mixed under N₂ atmosphere. The mixture is added with P(Ph)₃ (1.157 g, 4.41 mmoles) and Pd(OAc)₂ (0.33 g. 1.47 mmoles) while stirring, then refluxed under stirring for 20 hours. Upon completion of the reaction, the mixture is cooled to 40°C, added with H₂O, and the phases are separated. The organic phase is dried, filtered and evaporated to a thick oil which is directly used in the subsequent step.

44 g of the crude title compound are obtained; quantitative yield.

### EXAMPLE 5: 5-(1-Hydroxy-1-methyl-ethyl)-3-{2-[2-(1-methyl-1-phenylethyl)-2H-tetrazol-5-yl]-biphenyl-4-yl-methyl}-2-propyl-3H-imidazol-4-carboxylic acid (VI)

The compound of formula (**V**) of Example 3 (0.954 g, 1.72 mmol) is dissolved in MeOH (5 mL) and the resulting solution is added with a 2 M NaOH solution in H₂O (4.5 mL). After 5 hours the reaction is completed. MeOH is evaporated off under reduced pressure and the residue is extracted with Et₂O to remove any traces of the starting product. The aqueous phase is acidified to pH 2 with diluted HCI and extracted with AcOEt. The organic phase is dried over Na₂SO₄ and the solvent is evaporated off under reduced pressure.

610 mg of the title compound are obtained; yield: 67%.

¹H-NMR (CDCl₃) 1.02 (3H, br) 1.85 (6H, s) 2.08-1.93 (8H, m) 3.14 (2H, br) 5.73 (2H, s) 8.2-6.90 (13H, m).

### EXAMPLE 6: 5-(1-Hydroxy-1-methyl-ethyl)-3-{2-[2-(1-methyl-1-phenylethyl)-2H-tetrazol-5-yl]-biphenyl-4-yl-methyl}-2-propyl-3H-imidazol-4-carboxylic acid (VI)

The compound of formula (**V**) of Example 3 (44 g, 73.5 mmol) is dissolved in THF (120 mL) and the resulting solution is added with NaOH (5.9 g, 147 mmol) and H₂O (11.8 mL), refluxed under stirring until disappearance of the starting product, then added with CH₃COOH (9.7 g, 161.7 mmol) and H₂O (15 mL). After stirring for 15 min, the phases are separated. The organic phase is added with heptane (120 mL). The precipitated solid is filtered and dried in a static dryer under vacuum.

32 g of the title compound are obtained, yield: 77%.

Following the same procedure, starting from a compound of formula (**IV**), wherein R₄ is ethyl, the corresponding carboxylic acid is obtained.

### EXAMPLE 7: 5-(1-Hydroxy-1-methyl-ethyl)-3-{2-[2-(1-methyl-1-phenylethyl)-2H-tetrazol-5-yl]-biphenyl-4-yl-methyl}-2-propyl-3H-imidazol-4-carboxylic acid 5-methyl-2-oxo-[1,3]dioxol-4-yl-methyl ester (I; P=cumyl)

The compound of formula (**VI**) of Example 5 (610 mg, 1.081 mmol) is dissolved in DMF (4.2 mL). Na₂CO₃ (294 mg, 2.13 mmol) is added and the mixture is reacted for 30 minutes, after which medoxomil chloride (320 µL) is dropped therein. The reaction is kept under stirring at room temperature for 14 hours, then diluted with AcOEt and washed twice with H₂O. The organic phase is dried over Na₂SO₄ and the solvent is evaporated off under reduced pressure. The crude is subjected to purification by flash chromatography with 30 mm diameter column eluting with a hexane/ethyl acetate 1:1 mixture.

253 mg of the title compound are obtained; yield: 35%.

Following the same procedure, starting from the carboxylic acid of a compound of formula (**IV**), the corresponding compound of formula (**II**) is obtained.

### EXAMPLE 8: (5-methyl-2-oxo-1,3-dioxolen-4-yl)methyl-4-(1-hydroxy-1-methylethyl)-2-propyl-1-[4-[2-[2-(1-methyl-1-phenyl-ethyl)-2H-tetrazol-5-yl]phenyl]-phenyl]methylimidazole-5-carboxylate (I; P=cumyl)

A compound of formula (**II**) wherein X is Br (36.2 g, 73.5 mmol), 2-(2-cumyltetrazolyl)-phenylboronic acid (26 g, 84.5 mmol), K₂CO₃ (20.32 g, 147 mmol), THF (120 mL) and H₂O (3 mL) are mixed under N₂ atmosphere. The mixture is added with P(Ph)₃ (1.157 g, 4.41 mmol) and Pd(OAc)₂ (0.33 g, 1.47 mmol) while stirring, then refluxed under stirring for 20 hours. Upon completion of the reaction, the mixture is cooled to 25°C, added with H₂O and the phases are separated. The organic phase is dried, filtered and evaporated. The title compound (50 g) is obtained as a thick oil. The crude is directly used in the subsequent step.

### EXAMPLE 9: (5-methyl-2-oxo-1,3-dioxolen-4-yl)methyl-4-(1-hydroxy-1-methylethyl)-2-propyl-1-[4-[2-(tetrazol-5-yl)phenyl]-phenyllmethylimidazole-5-carboxylate (I; P=H) (olmesartan medoxomil)

The compound of Example 7 (55 mg, 0.08 mmol) is dissolved in MeOH (1 mL); the reaction mixture is cooled to 0°C and added with 37% HCI (2 mL). The reaction mixture is kept under stirring at room temperature for 4 hours, then evaporated under reduced pressure to a residue, taken up with a sodium acetate aqueous solution to pH of 4.5-5. The formed white precipitate is filtered.

40 mg of the title compound are obtained; yield: 89%.

¹H-NMR (MD₃OD) 0.95 (3H, t, J=7.4 Hz) 1.61 (6H, s) 1.64 (2H, sx) 2.03 (3H, s) 2.75 (2H, t J=7.7,) 5.03 (2H, s) 5.52 (2H, s) 5.76 (1H, s) 6.88 (2H, d, J=8.0 Hz) 7.09 (2H, d, J=8.0 Hz) 7.71-7.51 (4H, m).

## Claims

1. A process for the preparation of a compound of formula (**I**) 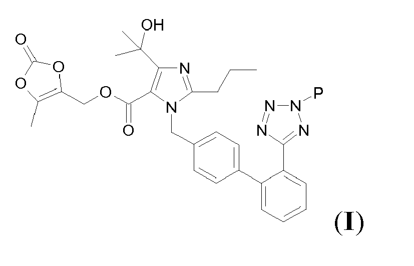 wherein P is a hydrogen atom or a 1-methyl-1-phenylethyl group, comprising:
a) the reaction of a compound of formula (**II**), or a salt thereof, 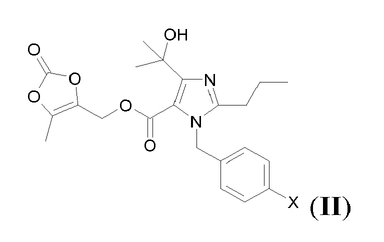 wherein X is a leaving group, with a synthon of formula (**III**), or a salt thereof 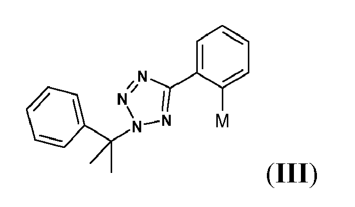 wherein
M is a -B(OR₁OR₂) group wherein each of R₁ and R₂ is, independently, hydrogen, C₁-C₈ alkyl, aryl, aryl-C₁-C₈ alkyl, or R₁ and R₂, taken together, form a -(CH₂)ₘ-V-(CH₂)ₙ group, wherein m and n, which can be the same or different, are 0 or 1, and V is NR₃ or C(R₃)₂ wherein R₃ is hydrogen, C₁-C₈ alkyl, aryl or aryl-C₁-C₈ alkyl; or M is a lithium or copper atom or a halogenated metal; in the presence of a catalyst, an organic ligand and a basic agent; or
b) the reaction of a synthon of formula (**III**), or a salt thereof, as defined above, with a compound of formula (**IV**), or a salt thereof, 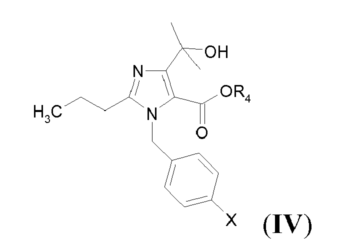 wherein X is a leaving group, and R₄ is C₁-C₈ alkyl, aryl or aryl-C₁-C₈ alkyl;
in the presence of a catalyst, an organic ligand and a basic agent;
to obtain a compound of formula (**V**) 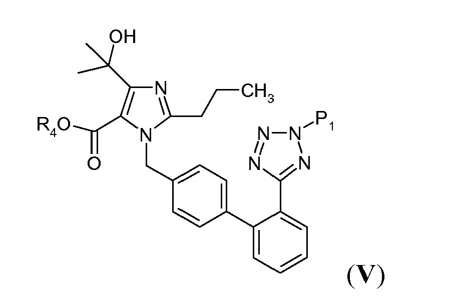 wherein P₁ is a 1-methyl-1-phenylethyl group and R₄ is as defined above;
b') the subsequent hydrolysis of the heterocycle ester group to a compound of formula (**V**), as defined above,
to obtain a compound of formula (**VI**), or a salt thereof, 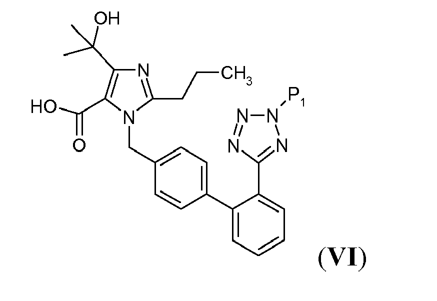 wherein P₁ is as defined above;
b") the subsequent reaction of a compound of formula (**VI**), as defined above, or a salt thereof, with a compound of formula (**VII**) 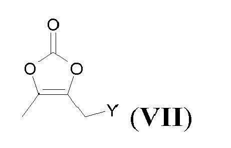 wherein Y is halogen; and, if desired, the conversion of a compound of formula (**I**) to another compound of formula (**I**).

2. The process as claimed in claim 1, wherein in a compound of formula (**III**) M is a -B(OR₁OR₂) group wherein each of R₁ and R₂ is, independently, hydrogen or C₁-C₄ alkyl.

3. The process as claimed in claim 1, wherein the catalyst is a Pd, Pt or Ni salt.

4. The process as claimed in claim 3, wherein the catalyst is a palladium (**II**) salt.

5. The process as claimed in claim 1, wherein the organic ligand is a phosphine.

6. The process as claimed in claim 1, wherein the organic base is a straight or branched tertiary amine and the inorganic base is potassium, sodium or cesium carbonate, sodium or potassium acetate, sodium or potassium hydroxide, sodium or potassium phosphate, or sodium or potassium hydrogen phosphate.

7. The process as claimed in claim 1, wherein the molar ratio of basic agent to compound of formula (**II**) or (**IV**), or a salt thereof, approximately ranges from 1:1 to 4:1.

8. The process as claimed in claim 1, wherein the reaction between a compound of formula (**III**), or a salt thereof, and a compound of formula (**II**), or a salt thereof, or of formula (**IV**), or a salt thereof, is carried out in the presence of an organic solvent, or in a mixture of two or three organic solvents; or in a mixture of one, two or three of them with water.

9. The process as claimed in claim 1, wherein the reaction is carried out between a compound of formula (**II**) or (**IV**), wherein X is bromine, and a synthon of formula (**III**), wherein M is a -B(OR₁OR₂) group wherein each of R₁ and R₂ is hydrogen; in the presence of palladium (**II**) acetate, triphenylphosphine, potassium carbonate, in a tetrahydrofuran-water mixture.

10. A compound of formula (**I**) 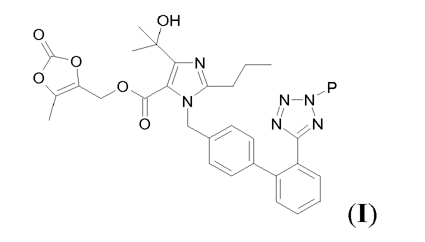 wherein P is cumyl.

11. A compound of formula (**II**), (**VI**) or (**XII**), or a salt thereof, or of formula (**V**) 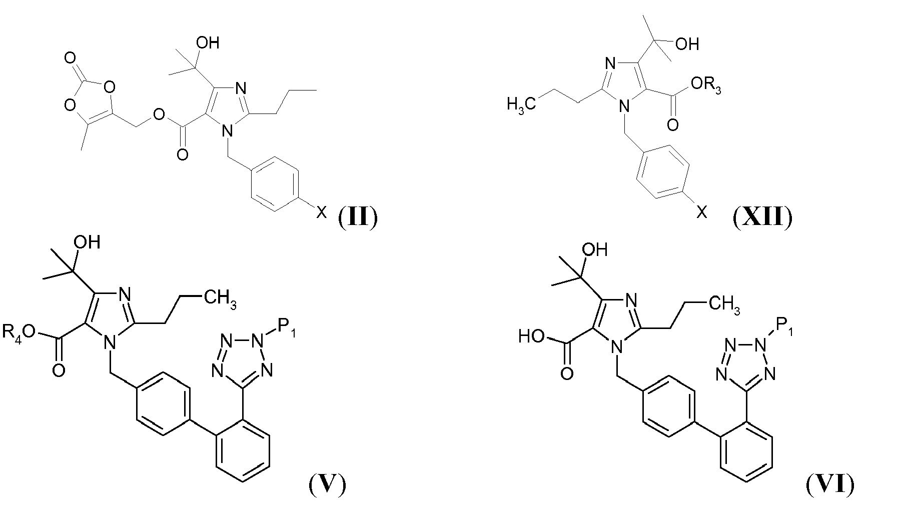 wherein R₃ is hydrogen, C₁-C₈ alkyl, aryl, aryl-C₁-C₈ alkyl; X is a leaving group; and P₁ is cumyl.
